# FASCICULE DE BREVET EUROPEEN

(11) **EP 0 406 103 B1**
(45) Date de publication et mention de la délivrance du brevet: **28.02.1996**
(21) Numéro de dépôt: 90401833.0
(22) Date de dépôt: 26.06.1990
(51) Int. Cl.: A01N 63/04, A01N 63/00

(54) **Compositions pesticides à base de microorganismes, leur procédé de préparation et leur application en agronomie**
Pestizide Zusammensetzungen auf der Basis von Mikroorganismen, Verfahren zu ihrer Herstellung und ihre Verwendung in der Landwirtschaft
Pesticidal compositions based on microorganisms, process for their preparation, and their use in agriculture

(30) Priorité: 27.06.1989 FR 8908553
(43) Date de publication de la demande: 02.01.1991
(73) Titulaire: INSTITUT NATIONAL DE LA RECHERCHE AGRONOMIQUE (INRA), F-75341 Paris Cédéx 07 (FR)
(72) Inventeur: Riba, Guy, F-93800 Epinay (FR); Goussard, Jacqueline, F-28150 Ouarville (FR); Durand, Alain, F-21240 Talant (FR); Desgranges, Catherine, F-21000 Dijon (FR)
(74) Mandataire: Phélip, Bruno

(56) Documents cités:
- EP-A- 0 226 394
- FR-A- 2 394 606
- FR-A- 2 494 717

## Description

La présente invention concerne de nouvelles compositions à base de champignons entomopathogènes , leur procédé de préparation et leur application en agronomie.

On connaît de nombreux composés chimiques de synthèse aux propriétés insecticides plus ou moins sélectives; bien que ces composés soient hautement efficaces, on souhaîte en général limiter leur utilisation,que ce soit pour une raison de coût ou de protection de l'environnement.

Dans la technique antérieure, on a déjà constaté que des microorganismes convenablement choisis et notamment certains champignons pouvaient, par un apport massif dans un agrosystème infesté,jouer le même rôle que des pesticides chimiques et limiter le développement d'insectes ravageurs de cultures, la multiplication de mauvaises herbes, ou d'autres champignons parasites de plantes.

Les insecticides biologiques sont plus spécifiques que la plupart des pesticides chimiques et n'induisent donc pas de phénomènes secondaires; en outre, ils ne sont pas toxiques pour les invertébrés et les vertébrés,et donc pour l'homme. Leur utilisation serait donc tout-à-fait avantageuse. Toutefois elle se heurte aux problèmes suivants; d'une part la prolifération des champignons entomopathogènes est limitée aux régions chaudes et humides, et dans les régions tempérées on considère actuellement qu'étant donné les doses élevées de champignons à utiliser, un tel procédé de lutte contre les insectes n'est pas économiquement applicable; d'autre part, ces germes sont particulièrement sensibles aux rayons ultraviolets.

Afin de pallier ces inconvénients des solutions techniques ont été proposées, notamment l'utilisation de supports solides. Ces supports sont d'ailleurs connus par l'homme de l'art, puisqu'ils ont été utilisés pour la préparation de compositions contenant des insecticides chimiques ou pesticides.Ainsi, le brevet GB 1002977 décrit plusieurs compositions contenant toutes comme support solide de la vermiculite ,chacune d'elles contenant aussi un pesticide donné , ainsi que divers agents tels que des agents mouillants.

Le brevet US 4530834 concerne un procédé de préparation de mycélium séché, qui comprend un certain nombre d'étapes , dont une étape de culture sur un milieu qui est défini par les inventeurs comme "tout milieu disponible commercialement" , une étape de traitement par un agent chimique protecteur afin d'augmenter la viabilité du mycélium ainsi qu'une étape d'incubation et de séchage . Il est précisé que les produits en vue d'une utilisation pour le contrôle des insectes sont moulus et mis sous la forme de poudre mouillable, et ensuite appliqués en pulvérisation sur les plantes.

Le brevet US 3013 946 concerne lui aussi un procédé de préparation de composition de champignons ayant un effet insecticide, par culture de microorganismes de la famille des Moniliales sur un support solide qui est la vermiculite. Il est précisé que la fermentation est effectuée pendant une période de 14 à 21 jours mais sans contrôle de l'état du champignon, notamment de la présence de spores.Le milieu de culture est desséché après fermentation et avant utilisation en épandage.

Parmi les ravages causés aux cultures par les insectes , les pyrales occupent une place de choix. Ainsi, les pyrales du maïs de la famille des pyralides :Ostrinia nubilalis et Ostrinia furnacalis. Plusieurs espèces de champignons entomopathogènes sont susceptibles d'attaquer ces pyrales; ainsi les oeufs de cet insecte sont très sensibles à des souches de Metarhizium anisopliae, tandis que les chrysalides meurent facilement de la muscardine rose à Paecilomyces fumosoroseus. Un autre champignon Beauveria bassiana est responsable de la muscardine blanche qui s'attaque aux larves de pyrales et existe à l'état endémique dans les populations de O. nubilalis.Comme la plupart des hyphomycètes , B.bassiana génère deux types de propagules infectieuses : des blastospores par bourgeonnement du mycélium,qui assurent la multiplication végétative du champignon uniquement présentes en culture submergée , et des conidies présentes en culture submergée et en culture à l'état solide .

Le brevet FR 2.394.6O6 concerne un procédé de préparation de microorganismes , dont B.bassiana, utilisables dans la lutte contre les maladies des plantes, consistant à mélanger une préparation aqueuse d'un microorganisme, avec une suspension d'argile, cet argile pouvant être de la montmorillonite ou de la bentonite.

Un article de 1979 de IGNOFFO et al. (J. Econ. Entomol.72; 562-565) décrit la production de Beauveria bassiana par fermentation en milieu liquide, suivie d'un séchage et d'un mélange avec du talc ou d'autres matières inertes , telles que la perlite, du kaolin,de la bentonite ou de l'amidon.

Le brevet EP-A-O 226 394 décrit un procédé de fermentation directe et en une seule étape de divers microorganismes sur des particules qui sont plus spécialement de la vermiculite . Le support particulaire ainsi traité peut être utilisé pour inoculer des cultures. Dans un mode de réalisation caractéristique , le procédé consiste à mélanger d'abord de la vermiculite , de la farine et de l'eau , à effectuer la stérilisation de ce mélange , à l'inoculer ensuite par un microorganisme , tel qu'un champignon, à effectuer consécutivement un stockage pour la maturation puis à réaliser un séchage ce qui aboutit à un produit particulaire pouvant être utilisé pour le traitement des cultures .

Le procédé décrit dans le brevet EP-A-O 226 394 ne mentionne aucunement le contrôle de la maturité de la culture du microorganisme,alors que ce paramètre est essentiel aussi bien pour permettre l'efficacité du produit dans les conditions d'épandage que pour optimiser la fabrication. Ce brevet antérieur ne décrit pas un produit dans lequel le microorganisme possède une aptitude à sporuler dans les conditions d'épandage . Il prévoit d'ailleurs une stérilisation du support. Si l'on se reporte aux exemples illustratifs donnés dans le brevet EP-A- O 226 394 , on constate que des durées de culture très importantes sont prévues : 45 jours dans l'exemple 1, 2 à 4 semaines dans les exemples 2 et 3. Pour l'homme du métier, de telles durées impliquent une absence de contrôle de la qualité et de la quantité des champignons dans les produits obtenus. Le procédé antérieur est d'autre part réalisé dans des conteneurs dont l'utilisation oblige d'effectuer la culture sur de faibles épaisseurs , sans aucune régulation . Le support préféré, tel qu'il est illustré dans les exemples de ce brevet antérieur , est la vermiculite, qui ne convient pas pour une application agronomique mécanisée.

Ainsi, le procédé décrit dans le brevet EP-A-O 226 394 ne permet pas d'aboutir , dans des conditions de culture industrielles , à un produit possédant l'aptitude à sporuler dans les conditions d'épandage.

Le brevet FR - A-2 298 951 concerne des produits se présentant sous forme de granulats, obtenus par extrusion,pouvant servir d'inoculant , de fertilisant ou dans d'autres applications analogues . Des microorganismes sont ajoutés à un support solide et le mélange est traité jusqu'à la forme solide désirée. Les microorganismes, essentiellement du genre Trichoderma sont incubés sur des grains concassés d'orge ou autres , dans des conteneurs fermés . Ce procédé ne permet aucun contrôle de la quantité de biomasse et de sa qualité .

Le brevet FR-A-2 298 951 n'enseigne pas l'utilisation de particules ou granulés précolonisés .

Toutes ces solutions au problème de la lutte biologique dans le cas de l'utilisation d'un champignon comme insecticide contre un insecte ravageur des cultures , présentent les mêmes inconvénients, à savoir qu'aucune d'entre elles ne présente une efficacité importante en ce qui concerne l'inhibition de la propagation ou de la croissance desdits insectes.

Le but de la présente invention est l'obtention d'un produit présentant une grande efficacité, une bonne stabilité,un faible coût de production,en particulier grâce à des durées de culture très réduites et une grande facilité d'utilisation.

L'invention a plus particulièrement pour objet un produit granulaire , précolonisé par des microorganismes,dont la maturité est contrôlée et trouvant application dans les traitements agronomiques .

L'objet de la présente invention est donc un produit de traitement agronomique à base d'au moins un microorganisme utile pour la lutte contre les ravageurs et les maladies des cultures ou pour la fertilisation des sols conforme à la revendication 1.

Ledit microorganisme peut être une bactérie permettant la fertilisation des sols, tel qu'un Rhizobium ,ou un champignon ectomycorrhizien tel qu'un Laccaria . Il peut être également un champignon entomopathogène, efficace contre un ou des insectes ravageurs des cultures, les insectes du sol (scarabéides et curculionides notamment ), les insectes piqueurs (cercopides notamment ) ou les insectes foreurs (pyrales notamment).

Ledit microorganisme peut aussi être un champignon pathogène vis-à-vis d'autres champignons, tel que notamment un Fusarium, non pathogène vis-à-vis des plantes mais pathogène vis-à-vis d'un Fusarium ravageur, ou un Trichoderma pathogène vis-à-vis de Sclerotinia, Rhizoctonia, Armillaria ou Botrytis .

Ledit microorganisme peut aussi avoir un effet pathogène vis-a-vis de nématodes, et il peut ainsi être un Arthrobotrys .

Un produit contenant un de ces microorganismes a notamment pour avantage, par comparaison avec les pesticides ou les engrais usuellement employés, de ne pas polluer l'environnement et de préserver la flore et la faune.

Le produit est épandable au champ directement après son obtention par culture directe et fermentation à l'état solide sur lesdites particules. Ceci est un avantage notable de l'invention: en effet il n'est pas nécessaire de moudre le produit à la sortie du fermenteur,ni de lui incorporer des additifs.

Selon un mode de mise en oeuvre de l'invention, le microorganisme est un champignon et il est en totalité sous forme mycélium au moment de l'épandage, auquel cas ledit produit est dit immature, ou le champignon est sous forme mycélium et spores, auquel cas ledit produit est dit mature. Ce contrôle de l'état de la population de champignons, et donc de l'efficacité du produit, est une caractéristique essentielle de l'invention .L'art antérieur, à la connaissance du demandeur, ne décrit pas de produits dont la composition en spores et en mycélium est contrôlée avec précision.Le produit selon l'invention peut aussi être composé d'un mélange des formes mature et immature .

Selon un autre mode de mise en oeuvre de l'invention, ledit support a une densité préférentiellement comprise entre 0,6 et 1.

Selon un autre mode de mise en oeuvre de l'invention le support est d'origine minérale ou organique, notamment composé de montmorillonite, d'attapulgite, ou de sépiolite. Le support doit être compatible avec une application mécanisée terrestre ou aérienne.

Selon un autre mode de mise en oeuvre de l'invention, ledit support a une capacité de rétention en eau d'au minimum 40%.

Selon un autre mode de mise en oeuvre de l'invention, lesdites particules ont une taille comprise entre 100 et 1000 µm et préférentiellement de 500 µm environ.

La présente invention a également pour objet un produit dans lequel ledit champignon est une souche de B.bassiana, notamment la souche déposée à la Collection Nationale de Cultures des Microrganismes sous le N°I 867 ou un mutant de cette souche.

Selon un mode de mise en oeuvre particulier de l'invention, ledit produit est mis sous une forme sèche pour le stockage et l'épandage.

La présente invention a d'autre part pour objet un procédé pour l'obtention d'un produit de traitement agronomique à base d'au moins un microorganisme, pour la lutte contre les ravageurs et les maladies des cultures ou pour la fertilisation des sols, caractérisé en ce qu'il comprend la culture directe par fermentation à l'état solide dudit microorganisme sur au moins un support solide en particules, insoluble dans le milieu de fermentation, et la récupération desdites particules , qui sont colonisées par le microorganisme, la maturité de la culture étant contrôlée pour permettre au microorganisme de sporuler dans les conditions d'épandage.

Ainsi l'association du microorganisme et des particules de support solide se fait sans aucune intervention d'un quelconque moyen ou agent physique et/ou chimique, ce qui est un avantage notable du procédé selon l'invention.

D'autre part les microorganismes précolonisent les particules durant la fermentation, ce qui assure une association plus efficace du microorganisme et des particules.

Selon un mode de mise en oeuvre du procédé de l'invention, le microorganisme est un champignon et on arrête la fermentation sensiblement au stade de l'obtention du mycélium, ce qui conduit à un produit dit immature ou au stade de l'obtention de mycélium et de spores, ce qui conduit à un produit dit mature.

L'arrêt de la fermentation en vue de la production d'un produit immature permet d'écourter de plusieurs jours la fermentation .

Selon un autre mode de mise en oeuvre du procédé selon l'invention,lesdites particules ont été ou non préalablement stérilisées. Ceci est également un avantage de l'invention car les procédés de l'art antérieur nécessitent, à la connaissance du demandeur , la stérilisation des supports. Cette amélioration du procédé de fermentation est donc très importante du point de vue de la production et du point de vue économique, car elle permet d'éviter une étape coûteuse en énergie .

Selon un autre mode de mise en oeuvre du procédé selon l'invention, le champignon utilisé est un champignon entomopathogène , efficace contre un ou des insectes ravageurs des cultures . Ce champignon peut être notamment B.bassiana, B.tenella, B. brognartii,ou Paecilomyces fumosoroseus.

Selon un autre mode de mise en oeuvre du procédé selon l'invention, ledit champignon est B. bassiana.Lorsque la fermentation a lieu durant 30 à 50 heures, et de préférence 48 heures, elle fournit un produit immature; lorsque la durée de fermentation est au minimum de 90 heures, on obtient alors un produit mature.

On notera aussi que le procédé de l'invention peut être mis en oeuvre avec des épaisseurs élevées de support au cours de la fermentation,atteignant plusieurs dizaines de cm, ce qui est favorable pour la production industrielle ainsi que pour le contrôle de la maturité de la culture.

L'invention a d'autre part pour objet un procédé de lutte biologique ,contre les maladies et les ravageurs des végétaux ,caractérisé en ce que l'on épand une quantité efficace du produit défini précédemment , notamment à base de champignons enthomopathogènes , sur les végétaux à traiter, la maladie à traiter pouvant être par exemple la pyrale du maïs qui est due à O. nubilalis, mais aussi d'autres pyrales ou d'autres maladies des végétaux.

L'invention a de plus pour objet un procédé de fertilisation des sols , caractérisé par le fait qu'on épand une quantité efficace du produit fertilisant à base de microorganismes précédemment défini.

La description qui suit donne à titre illustratif et non limitatif des exemples d'applications de l'invention; en référence aux dessins annexés sur lesquels :
Fig.1 est un schéma de réacteur-fermenteur pouvant être utilisé pour obtenir le produit de l'invention.
Fig.2 est un graphique représentant l'évolution de paramètres biochimiques en fonction du stade de la culture suivant le procédé décrit dans l'exemple 1;
Fig.3 est un graphique illustrant les résultats des essais rapportés à l'exemple 4.

### EXEMPLE 1

### Production des produits selon l'invention

### a) - Préparation de l'inoculum

Un milieu liquide semi-synthétique A (tableau 1) est ensemencé au moyen de conidies de B. bassiana, issues d'une culture sur milieu gélosé semi-synthétique B (tableau 2) âgée de 5 jours, à un taux de 10⁶ conidies/ml. Ce milieu est incubé à 25°C, en fioles sur table d'agitation ou en fermenteur liquide en fonction des quantités de culture à inoculer.

Après 72 heures de culture, le milieu A contient entre 1 et 2.10⁸ blastospores/ml.

**TABLEAU 1**

| Composition du milieu A | |
|---|---|
| Liqueur de macération de céréales | 20,0g |
| Saccharose | 30,0g |
| KNO₃ | 5,0g |
| KH₂ PO₄ | 6,8g |
| CaCO₃ | 2,0g |
| MgSO₄ 7H₂O | 0,1g |
| Eau permutée | 1 l |

**TABLEAU 2**

| Composition du milieu B | |
|---|---|
| Glucose | 10,0g |
| Extrait de levure | 5,0g |
| KH₂ PO₄ | 0,4g |
| Na₂ HPO₄, 12H₂O | 1,4g |
| Mg SO₄, 7H₂O | 0,6g |
| KCl | 1,0g |
| NH₄ NO₃ | 0,7g |
| Chloramphénicol | 0,5g |
| Agar | 15,0g |
| Eau permutée | 1 l |

### b) - Préparation du milieu de culture à l'état solide

Le milieu de culture utilisé comprend deux phases:
- une phase solide constituant le support granulé;
- une phase liquide constituant le substrat nutritif du micro-organisme et imbibant la phase solide.

Le support peut être préalablement stérilisé (3h à 120°C) ou non, un procédé stérile ne s'imposant pas puisque peu de contaminants se développent dans les conditions de culture utilisées et que des résultats satisfaisants sont obtenus.

Ce support est imprégné par un des milieux nutritifs C, D ou E (tableaux 3, 4 et 5 ); ces milieux nutritifs C ou D ou E stérilisés (30 minutes à 115°C), étant additionnés de l'inoculum à raison de 3.10⁶ blastospores/ml de milieu C, D ou E.

L'imprégnation et l'homogénéisation peuvent être effectuées dans un appareil type pétrin de boulangerie ou dans le réacteur lui-même, de façon à obtenir un taux de matière sèche d'environ 60% (poids du support/poids humide). On obtient dans ces conditions un taux d'inoculation d'environ 2.10⁶ blastospores/g de matière sèche de support. Selon le cas la souche est incubée entre 18 et 27°C.

**TABLEAU 3**

| Composition du milieu C | |
|---|---|
| Saccharose | 132,0g |
| Non Sucre résiduel * | 27,Og |
| Eau permutée | 1 l |

| | |
|---|---|
| * Le Non Sucre Résiduel est un sous produit de l'extraction du sucre composé principalement de matières azotées et commercialisé par la Générale Sucrière. | |

**TABLEAU 4**

| Composition du milieu D | |
|---|---|
| Solulys ** (Société Roquette) | 150 g |
| Eau permutée | 1 l |

| | |
|---|---|
| ** Le solulys est un sous produit de l'extraction et du raffinage de l'amidon de maïs, qui contient notamment des acides aminés et de l'acide lactique. | |

**TABLEAU 5**

| Composition du milieu E | |
|---|---|
| Saccharose | 124,0g |
| Solulys | 38,5g |
| Eau permutée | 1 l |

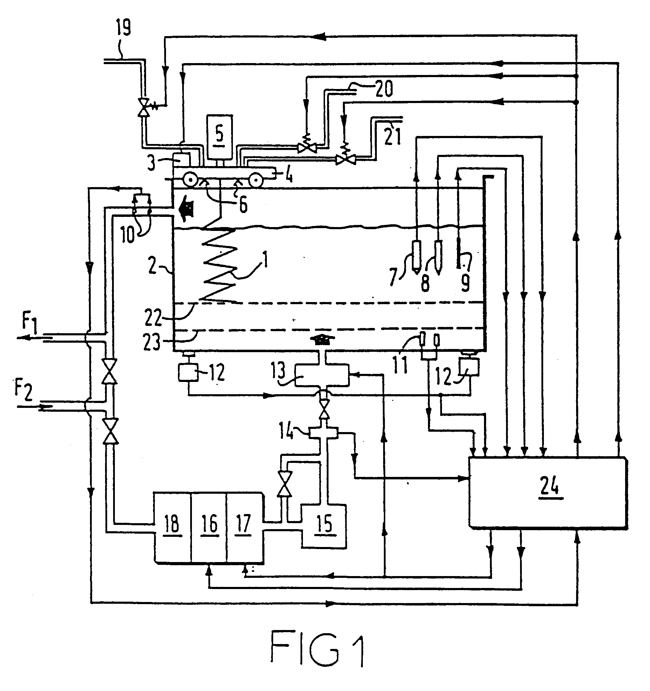

L'ensemble du mélange homogène est transféré dans le réacteur (Figure 1) , si le mélange n'y est pas directement effectué.

La Figure 1 représente le réacteur et son dispositif de régulation et de contrôle.

Le réacteur est composé d'une cuve (2), remplie d'un milieu semi-solide,dans laquelle trempent une ou plusieurs hélices de retournement (1) portées par un chariot mobile (4) et entraînées par un moteur (5).

Le chariot (4) est mû par un moteur d'avancement (3) et porte de plus une rampe de pulvérisation (6) alimentée par les tubulures d'arrivée de solutions (19, (20) et (21).

Au voisinage du fond du réacteur et parallèlement à celui-ci se trouvent une plaque perforée supérieure (22) constituant un tamis grossier servant à retenir la couche de milieu de culture, qui peut atteindre 0,50 à 1 mètre, et une plaque perforée inférieure (23) formant un tamis plus fin et permettant la distribution de l'air de façon égale à travers l'enceinte de la cuve (2). Des deux tamis peuvent être remplacés avantageusement par un unique tamis constitué d'un maillage tressé en acier inoxydable (tôle DYNAPORE), jouant à la fois le rôle de diffuseur d'air et de support du milieu de culture.

L'air pénètre dans le dispositif de fermentation dans le sens indiqué par la flèche F₂ et en sort dans le sens indiqué par la flèche F₁. Il est d'abord filtré par le filtre (18), puis, si nécessaire, refroidi à l'aide de la batterie froide (16) ou chauffé à l'aide de la batterie chaude (17) et humidifié dans l'humidificateur (13).

La circulation de l'air est assurée par le ventilateur (15); le débit est de l'ordre de 7,5 1.mn⁻¹-kg de matière sèche ⁻¹.

Le dispositif de mesure des conditions de culture dans le réacteur est constitué d'une sonde à oxygène (7), d'une sonde de pH (8) et d'une sonde de température (9). La mesure de la température et de l'humidité de l'air est assurée par les sondes(11) à l'entrée et en sortie (1O) de réacteur Des jauges de contrainte en compression (12)permettent de mesurer la masse en culture dans le réacteur et la mesure du débit d'air est effectuée à l'aide du débit-mètre.

Le contrôle de la fermentation et la régulation du réacteur sont assurés par le micro-calculateur (24) qui reçoit les informations fournies par la sonde à oxygène (7), la sonde de pH (8), la sonde de température (9), les sondes de température et d'humidité (10) et (11), les capteurs de contraînte en compression (12), le débit mètre (14), et qui, en fonction de ces informations agit sur le moteur d'avancement du chariot (3), l'humidificateur (13), le ventilateur (15), les batteries froide (16) et chaude (17), les vannes contrôlant les débits des tubulures d'arrivée de so lutions (19), (20) et (21).

Durant la culture, la température est maintenue à 25°C environ et l'humidité de la culture à 40% environ par ventilation d'un air chaud et humide.

### d) - Contrôle de l'état de la culture en cours de production .

Il est important de pouvoir suivre en cours de culture l'évolution de la production, c'est-à-dire le stade auquel se trouve le champignon (mycélium ou spore ) afin de décider ou non de l'arrêt de la culture.

Suivant le stade de la culture, il faut pouvoir estimer d'une part la croissance mycélienne, d'autre part la conidiogénèse. Le suivi de la conidiogénèse s'effectue ponctuellement par une méthode standardisée de dénombrement des conidies .

### Methode de dénombrement des conidies

On pèse exactement environ 1g de culture et on introduit cette quantité dans un flacon contenant 5O ml de Tween 8O à 1 % et environ 3Og de billes de verre d'un diamètre de 4 mm . Les flacons sont mis en agitation sur agita teur rotatif pendant 2O mn.

Une dilution au dixième de la suspension ainsi obtenue est effectuée dans une solution de bleu de méthylène Après une heure d'incubation à température ambiante, les numérations sont réalisées sur cellule de Malassez.

Composition de la solution de bleu de méthylène :

| | |
|---|---|
| Bleu de méthylène | O,6 g |
| KH₂ PO₄ | 81,3 g |
| Na₂HPO₄, 12H₂O | 5,7 g |
| Eau | 1,O l |

### Estimation de la croissance mycélienne

L'estimation directe de la croissance mycélienne ne peut s'effectuer en ligne . Cependant , il est possible de suivre automatiquement l'évolution de la respiration , ce qui donne une parfaite image de l'état physiologique du microorganisme .

La mesure de la respiration fongique est la seule méthode qui permet à l'heure actuelle d'effectuer un suivi en temps réel des cultures . Les gaz sont pompés en sortie de fermenteur , asséchés au moyen d'un "Hygrostop" (Hartman et Braun). Leur composition est déterminée au moyen d'un analyseur de gaz à infra-rouge type URAS 3G (Hartman et Braun) pour le CO₂ et d'un analyseur paramagnétique d'oxygène , type Magnos 3 (Hartman et Braun). L'ensemble est géré par un microordinateur permettant de calculer en ligne différents paramètres: - le CPR ou taux de production de CO₂
- l'OUR ou taux de consommation d'oxygène.

Ces deux paramètres sont calculés et exprimés en mol de CO₂ ou O₂ par heure et par gramme de matière sèche.

Par ailleurs des analyses effectuées en différé permettent de suivre les évolutions des taux de glucosamine et d'ergostérol .

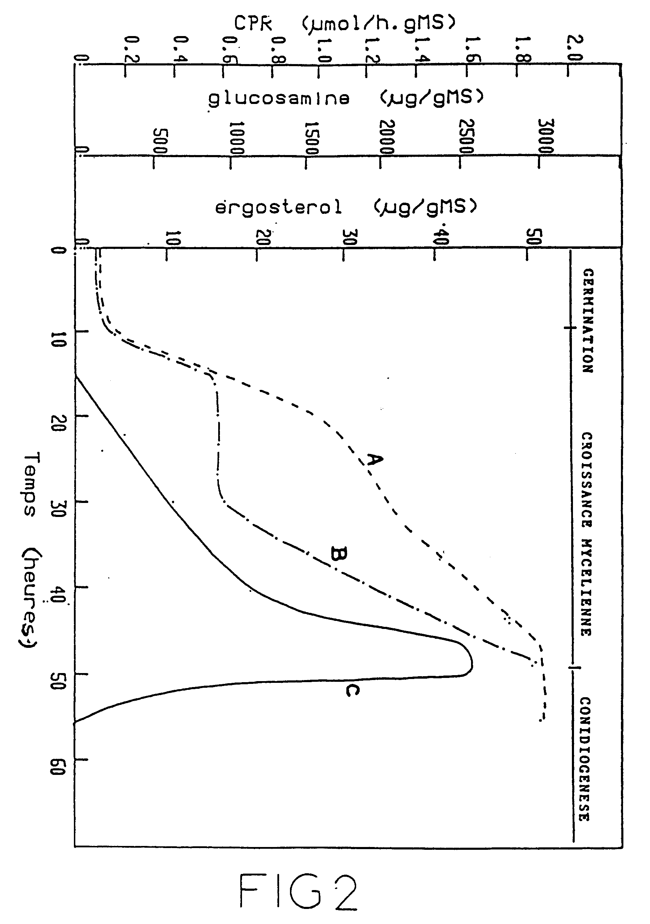

Un exemple de résultat obtenu lors d'une culture sur milieu C est représenté sur la figure 2 dans laquelle on a porté en abscisses le temps de culture, à partir de l'inoculation du fermenteur, et en ordonnées le CPR, le taux de glucosamine et le taux d'ergostérol . La courbe A représente l'évolution du taux de glucosamine, la courbe B l'évolution du taux d'ergostérol, la courbe C représente l'évolution du CPR.

### e)Contrôle de la quantité de produit obtenue en fin de fermentation .

Le microgranulé porte à la fois du mycélium et des conidies . Il est difficile de séparer quantitativement d'une part les deux formes l'une de l'autre et d'autre part le mycélium du support. Seules des méthodes de mesure indirectes sont réalisables . Elles consistent à doser,après extraction,des constituants biochimiques du mycélium et des spores ,la chitine et l'ergostérol.

### La chitine et l'ergostérol.

Ces méthodes , bien que ne permettant pas de contrôler en ligne la maturité du produit, puisque trop longues sont tout à fait satisfaisantes pour caractériser le produit en fin de fermentation. Le dénombrement des conidies est effectué en parallèle,selon la méthode de numération dé crite précédemment en d), Cette méthode n'est applicable qu'à partir de 72 heures de culture , car avant le taux de spores est trop faible pour effectuer un dénombrement.La biomasse totale, est estimée par dosage de la chitine et de l'ergostérol.

### Dosage de la chitine

La chitine, constituant de la paroi cellulaire, est hydrolysée en milieu acide et à chaud en N acétyl glucosamine (SAKURAI et al.(1977) Agric. Biol. Chem.41, 4, 619-624) .La glucosamine réagit après désamination, avec un réactif coloré, le méthyl 2-benzothiazone hydrazone (MBTH) . L'intensité de la coloration bleue est proportionnelle à la quantité de glucosamine (TSUJI et al. 1969 Chem. Pharm. Bull., 17,7,15O5-151O, RIDE et DRYSDALE DRYSDALE,1972,Physiological Plant Pathology, 2, 7,15).

L'hydrolyse est effectuée de la manière suivante:
l'échantillon à doser est préalablement lavé à trois reprises avec une solution de KCl à 1 mol.l⁻¹ afin d'éliminer les interférences dues au milieu nutritif résiduel puis on pèse précisément 1g d'échantillon lavé, on ajoute 1O ml HCl concentré, on laisse à température ambiante pendant 16 heures , on ajoute 4O ml d'eau permutée et enfin on autoclave cette solution 2 h à 13O°C.

Le dosage de la chitine est effectué en deux étapes :

### - Neutralisation de l'hydrolysat

1 ml d'hydrolysat est neutralisé par addition de 2,5 ml de NaOH à 1 mol.l⁻¹ et de 1,5 ml d'eau permutée.

### - Dosage

A 1 ml de la solution obtenue ci-dessus sont ajoutés 1 ml de KHSO₄ à 5% et 1 ml de NaNO₂ à 5%. On attend 15 minutes en agitant les tubes puis on ajoute 1 ml de NH₄ SO₃ NH₂ à 12,5%, on agite pendant 5 minutes ; on ajoute 1 ml de MBTH à O,5 % et on porte au bain-marie pendant 5 minutes après quoi on refroidit et ajoute 1 ml de FeCl₃ à O,5 % ; on attend 3O minutes, puis on lit à 65O nm.

La quantité de glucosamine des échantillons est déterminée par rapport à une gamme de N acétyl glucosamine (O à 3O ug/ml) réalisée simultanément.

Les résultats sont exprimés en ug de glucosamine/g de matière sèche d'échantillons.

### Dosage de l'ergostérol

L'ergostérol (constituant de la membrane cellulaire ) est extrait du produit brut :
- par saponification du produit, puis par séparation liquide/liquide, les stérols sont extraits et séparés des acides gras et de toutes substances insaponifiables.
- par élution en chromatographie liquide haute performance, l'ergostérol est séparé des autres stérols.
- par spectrophotométrie d'absorption dans l'UV, la quantité d'ergostérol est déterminée précisément. Effectivement , l'ergostérol possède une longueur d'onde d'absorption spécifique à 282 nm et la surface du pic obtenu sur le chromatogramme est proportionnelle à la quantité d'ergostérol présente. (SEITZ et al. 1979,Phytopathology, 69, 11, 12O2-12O3, SEITZ et al. 1977 Cereal Chem.54, 6, 12O7-1217.KAMINSKI et al. 1983 Fat Science Proc. 16th, 1st Congress, Budapest).

L'extraction est effectuée pratiquement de la manière suivante:
on pèse précisément une masse d'échantillon dans un ballon (quantité suffisante pour que l'extrait contienne O,1 à 3O ug/1O ul). On ajoute 1OO ml de méthanol et 5O ml de potasse alcoolique (KOH 2Og - alcool éthylique 5O ml). On saponifie le tout sous reflux , pendant 3O min puis on centrifuge 1O min. à 6OOO t.min.⁻¹. On mélange le surnageant avec 1OO ml d'eau et 1OO ml d'éther de pétrole. On laisse le tout dans une ampoule à décanter, puis on récupère la phase éther (phase supérieure) et on l'évapore. Le résidu sec est repris par 1O ml de dichlorométhane.

L'ergostérol est dosé dans l'extrait ainsi obtenu par chromatographie liquide haute performance sur une colonne garnie de silice Lichrososorb SI 6O de 5 um de granulométrie (Merck). La phase mobile est un mélange de chlorure de méthylène -isopropanol (99,5/O,5 V/V).

Les résultats sont exprimés en µg d'ergostérol par gramme de matière sèche .

### f)Contrôle de la viabilité du produit en fin de fermentation

Les méthodes de dosage décrites en e) permettent de quantifier la biomasse totale en fin de procédé, mais non de vérifier la viabilité du produit, d'une part en fin de culture, d'autre part après une période de stockage donnée . Habituellement, la réalisation d'un test de germination en boîtes de Pétri permet de déterminer la viabilité d'un produit sporulé, le résultat exprimé étant le nombre de conidies germées donnant naissance à une colonie par rapport au nombre total de conidies mesuré à la cellule de Malassez .

Etant donné que le produit microgranulé contient essentiellement du mycélium et qu'ainsi, aucune corrélation entre la biomasse mycélienne et le nombre de colonies apparues n'est possible, il a été mis au point une méthode d'analyse permettant d'estimer la viabilité du produit . Il s'agit de mesurer l'aptitude du mycélium à sporuler.

L'échantillon de culture séchée est humidifié et incubé . Pendant l'incubation, le mycélium poursuit sa croissance qui avait été stoppée par le séchage et sporule. Après 144 h d'incubation totale (incubation pendant la culture et pendant le test de viabilité), le taux de conidies a atteint un maximum et reste constant. Pour des cultures réalisées toujours dans les mêmes conditions, le taux maximum de conidies obtenues est toujours le même . Il suffit donc de déterminer le taux de conidies après 96 h d'incubation. La valeur obtenue est une indication de la qualité du produit.

### Méthode de mesure

Dans une fiole Erlenmeyer de 5OO ml, sont introduits 5Og d'une culture séchée.

Les 5O g sont humidifiés avec 22 ml d'eau stérile afin d'atteindre un taux de matière sèche compris entre 6O et 7O%. Les fioles sont ensuite incubées dans une salle climatisée à 25°C pendant 96 heures.

Après cette période d'incubation, le taux de conidies est déterminé comme décrit en d).

### g) - Stockage

Les compositions granulées selon l'invention sont stockées après séchage dans une atmosphère dont l'humidité est contrôlée sous gaz inerte (azote, gaz carbonique) ou encore sous vide de préférence à une température comprise entre 5 et 30°C.

### EXEMPLE 2

### Comparaison en champ de l'efficacité des microgranulés obtenus à différents stades de maturation

Des préparations de granules de montmorillonite de diverses maturités correspondant à des temps d'incubation de 48, 72, 96 et 120 h ont été préparées selon le mode opératoire général décrit en référence à l'exemple 1 et comparées (tableau 6).

L'efficacité a été estimée dans un essai de type blocs complets à quatre répétitions. Chaque parcelle est infestée hebdomadairement durant 3 semaines . A chaque infestation on introduit une ooplaque de pyrale par plante.

L'efficacité est exprimée en pourcentage de réduction par rapport au témoin dont l'effectif est de 1,9 chenille par plante.

Les granulés obtenus après fermentation de 48, 72 et 96 heures sont considérés comme immatures, tandis que ceux obtenus à 120 heures sont considérés comme matures.

Le mélange est composé de 70% de granules de 48 heures et de 30% de granules matures.

Il ressort de cette étude que des microgranulés immatures, séchés avant la conidiogenèse sont très fortement infectieux sur le terrain sans qu'il y ait le moindre effet retard.

Il est important de noter que ces microgranulés immatures ne contiennent pas de propagules infectieuses (conidies), mais qu'ils sont capables d'en générer après l'application au champ.

**TABLEAU 6**

| Etude des granules à maturité contrôlée | | | |
|---|---|---|---|
| Maturité en heures d'incubation | Dose d'application kg/ha | EFFICACITE | |
| | | % de réduction nombre d'attaques | % de réduction nombre de larges |
| 48 | 25 | 87,3 | 9O |
| 72 | 25 | 75,9 | 9O,8 |
| 96 | 25 | 91,7 | 94 |
| 12O | 25 | 68,2 | 79,2 |
| mélange | 25 | 88,1 | 93,3 |

### EXEMPLE 3

### Comparaison en champ de l'efficacité du produit selon l'invention et des produits issus d'autres technologies (formulations liquides ou solides).

Une préparation constituée d'un mélange de granulés, tel que défini dans l'exemple 2 (7O% de granules de 48 heures et 3O% de granules matures de 12O heures) , et obtenue selon le mode opératoire de l'exemple 1 a été testée en comparaison avec des formulations préparées selon des procédés décrits dans l'art antérieur.

Les essais ont été réalisés sur des champs de maïs.

Les formulations liquides testées sont les poudres mouillables suivantes:
- une suspension de conidies enrobées dans l'argile selon le procédé décrit dans la demande de brevet FR-A-2 394 606;
- une suspension de conidies enrobées dans des granules d'alginate additionnés ou non d'éléments nutritifs;
- une suspension mycélienne obtenue selon le procédé décrit dans le brevet US-4.530.834.

Ces suspensions liquides sont appliquées à la dose de 10¹³ conidies ou de 10O g de mycélium (dans 2OO l) par hectare .

Les résultats des essais sont portés dans le tableau 7 .

L'efficacité a été estimée dans un essai de type blocs complets à quatre répétitions. Chaque parcelle est infestée hebdomadairement durant 3 semaines. A chaque infestation on introduit une ooplaque de pyrale par plante.

L'efficacité est exprimée en pourcentage de réduction par rapport au témoin dont l'effectif est de 1,9 chenille par plante.

Ces résultats montrent que la formulation granulée obtenue selon l'invention est très nettement plus efficace que les poudres mouillables testées, quant à la réduction du nombre d'attaques des plantes et quant à la réduction de la population larvaire.

Les formulations solides testées sont de deux types: fixation ou adsorption (US-4.263.036) de propagules (conidies ou blastospores) infectieuses sur des microgranulés imprégnés ou non d'un milieu nutritif. Un tel procédé est précisé dans le brevet GB-1.002.977 pour des produits chimiques. Les résultats des expérimentations sont portés dans le tableau 8.

Les conidies ont été adsorbées en suspension dans du milieu complet à base de glucose (1,5%) et d'extrait de levure (1,5%).

L'efficacité a été calculée de la même manière que précédemment.

Ces résultats montrent une très nette augmentation de l'efficacité de réduction de l'attaque du maïs par O. nubilalis et de la réduction de la population larvaire d'O.nubilalis lors du traitement par le produit précolonisé selon l'invention.

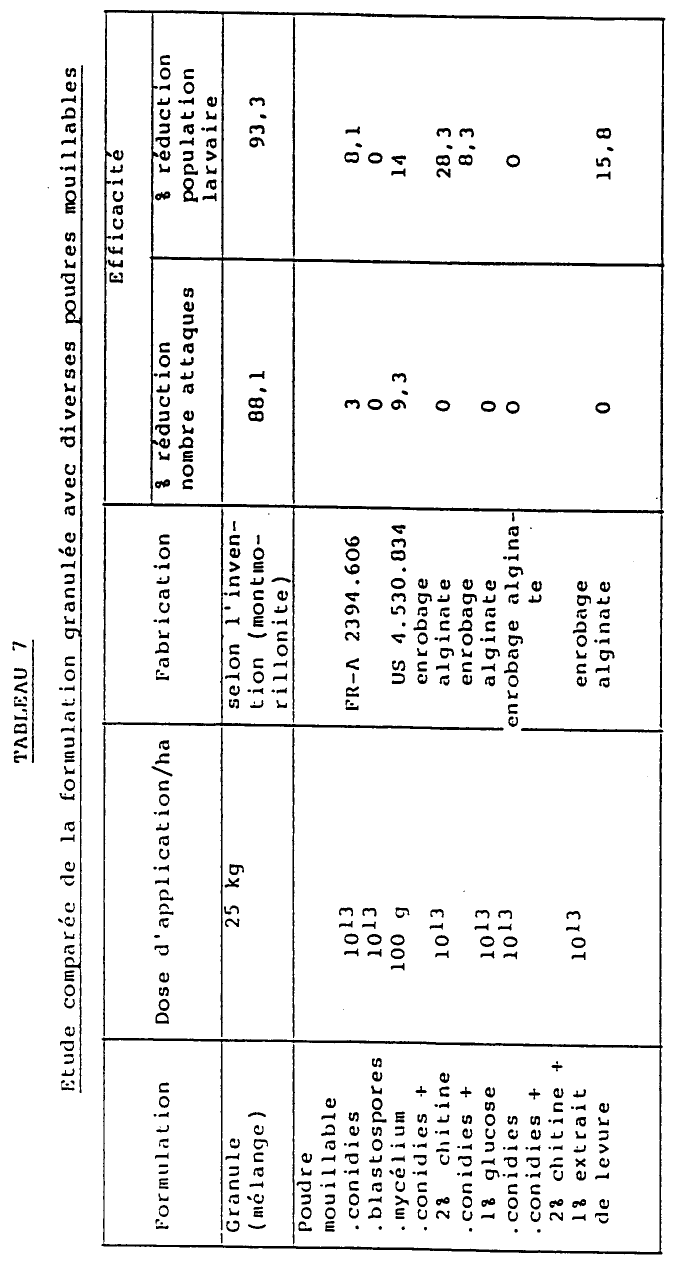

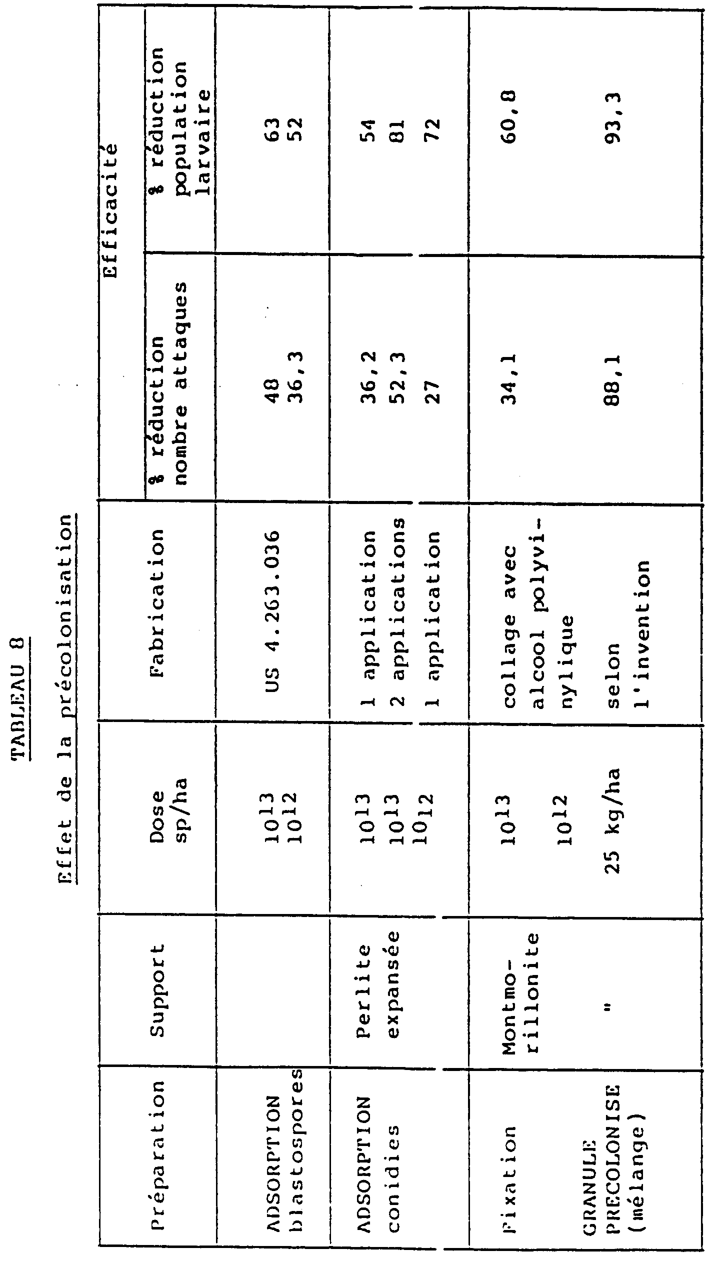

### EXEMPLE 4

### Comparaison du produit selon l'invention et des formulations chimiques de référence

Une préparation constituée d'un mélange de granules, tel que défini dans l'exemple 2 (7O% de granules de 48 heures et 3O% de granules de 12O heures), et obtenue selon le mode opératoire de l'exemple 1 a été testée en comparaison avec des insecticides de référence (tableau 9).

Les insecticides chimiques de référence sont le granulé DURSBAN G qui est un chlorpyriphos (fabriqué par la Quinoléine )appliqué à la dose de 25 kg/ha et le DECIS G (Société Procida) appliqué à la dose de 25 kg/ha.

**TABLEAU 9**

| Efficacité comparée du produit de l'invention et des insecticides chimiques de référence | | | |
|---|---|---|---|
| Produit | Dose d'application kg/ha | Efficacité | |
| | | % de réduction attaques | % de réduction population larvaire |
| GRANULE (mélange sur montmorillonite) selon l'invention | 25 | 88,1 | 93,3 |
| DECIS G | 25 | 9O | 8O |
| DURSBAN G | 25 | 91,3 | 9O,8 |

Les efficacités de réduction des attaques et de réduction de la population larvaire ont été calculées de la même manière que dans l'exemple précédent. Ces résultats ne mettent pas en évidence de différences significatives entre les actions du produit selon l'invention et des deux insecticides testés, ce qui montre que le produit selon l'invention a un effet au moins comparable à ceux de ces deux produits.

Les rémanences au champ de l'effet insecticide des produits matures et immatures de l'invention, d'une part et de la formulation chimique DECIS G d'autre part , ont été testées.

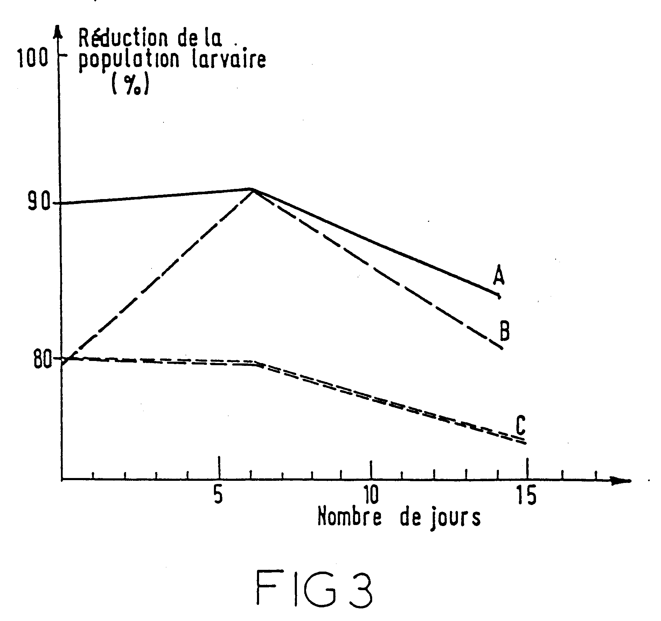

Sur la figure 3 on a porté en abscisses le temps après le début de l'expérience, exprimé en jours, et en ordonnées la réduction de la population larvaire, exprimée en pourcentage.

Les courbes A, B et C représentent respectivement l'évolution de l'effet insecticide des granulés immatures selon l'invention, des granulés matures selon l'invention, et des granulés de DECIS G.

Ces résultats montrent que la rémanence au champ de l'effet insecticide du produit de l'invention est comparable à celle de l'insecticide chimique DECIS-G,qui est un produit de référence.

### EXEMPLE 5

### Comparaison de l'efficacité de supports granulés

Des préparations obtenues par le mode opératoire général décrit à l'exemple 1 par fermentation jusqu'au stade mature de Beauveria bassiana sur des supports différents ont été testées pour leur efficacité de réduction des attaques et de réduction de la population larvaire. Les résultats sont résumés dans le tableau 1O ;les efficacités de réduction ont été calculées comme dans les exemples précédents.

Ces résultats montrent que la perlite expansée et la vermiculite permettent une bonne production des germes en ce qui concerne les efficacités de réduction des attaques et du nombre de larves.

Mais ces deux supports sont incompatibles avec l'application mécanisée. La chamotte et l'artal présentent une moindre efficacité. La montmorillonite est le support qui a fourni les meilleurs résultats pour la production et l'efficacité d'application.

L'invention a été illustrée précédemment dans son application particulière au champignon B.bassiana. Il est bien entendu cependant qu'elle n'est aucunement limitée à ce type particulier de champignon.

**TABLEAU 1O**

| Comparaison de l'efficacité de supports granulés | | | | | |
|---|---|---|---|---|---|
| SUPPORT | DOSE D'APPLICATION kg/ha | CARACTERISTIQUES | | EFFICACITE | |
| | | taille | densité | % réduction attaques | % réduction population larvaire |
| | | en mm | | | |
| Perlite expansée et vermiculite | 25 | 1-2 | O,1 | 86,8 | 9O |
| | 2,5 | - | - | 79,6 | 86,7 |
| Montmorillonite (granules matures) | 25 | O,5-1 | O,6 | 68,2 | 79,2 |
| Chamotte (mature) | 25 | O,5-1 | 1,35 | 64,2 | 7O,O |
| Artal (mature | 25 | O,5-1 | 1,2 | 38,4 | 7O,O |

## Revendications

1. Produit de traitement agronomique à base d'au moins un microorganisme utile pour la lutte contre les ravageurs ou les maladies des cultures ou pour la fertilisation des sols, caractérisé en ce qu'il comprend des particules formées d'un support solide portant le microorganisme, compatible avec une application mécanisée, et en ce qu'il est épandable au champ directement après son obtention par culture directe et fermentation à l'état solide sur lesdites particules, lesdits microorganismes précolonisant les particules durant la fermentation, la maturité de la culture du microorganisme étant contrôlée.

2. Produit selon la revendication 1, caractérisé en ce que le microorganisme est un champignon.

3. Produit selon la revendication 2, caractérisé en ce que le champignon est en totalité sous forme mycélium au moment de l'épandage, auquel cas ledit produit est dit immature.

4. Produit selon la revendication 2, caractérisé en ce que le champignon est sous forme mycélium et spores, auquel cas ledit produit est dit mature.

5. Produit selon la revendication 2, caractérisé en ce qu'il est composé d'un mélange des formes mature et immature.

6. Produit selon l'une quelconque des revendications 1 à 5, caractérisé en ce que ledit support a une densité préférentiellement comprise entre 0,6 et 1.

7. Produit selon l'une quelconque des revendications 1 à 6, caractérisé en ce que ledit support est d'origine minérale ou organique.

8. Produit selon la revendication 7, caractérisé en ce que ledit support est de la montmorillonite.

9. Produit selon la revendication 7, caractérisé en ce que ledit support est de l'attalpulgite.

10. Produit selon l'une quelconque des revendications 1 à 9, caractérisé en ce que ledit support a une capacité de rétention en eau d'au minimum 40%.

11. Produit selon l'une quelconque des revendications 1 à 10, caractérisé en ce que lesdites particules ont une taille comprise entre 100 et 1000 microns et préférentiellement ont une taille de 500 microns environ.

12. Produit selon l'une quelconque des revendications 2 à 11 caractérisé en ce que le champignon utilisé est un champignon entomopathogène, efficace contre un ou des insectes ravageurs des cultures.

13. Produit selon l'une quelconque des revendications 2 à 12, caractérisé en ce que le champignon est Beauveria bassiana.

14. Souche de Beauveria bassiana déposée à la Collection Nationale de Cultures de Microorganismes sous le N°I 867 et utilisée dans le produit selon l'une quelconque des revendications 1 à 13.

15. Souche de Beauveria bassiana caractérisée en ce qu'elle résulte d'une ou plusieurs mutations de la souche suivant la revendication 14.

16. Produit selon la revendication 13, caractérisé en ce que la souche Beauveria bassiana est une souche selon l'une des revendications 14 et 15.

17. Produit selon l'une quelconque des revendications 1 à 13 et 16, caractérisé en ce qu'il est sous forme sèche pour le stockage et l'épandage.

18. Procédé pour l'obtention d'un produit de traitement agronomique à base d'au moins un microorganisme pour la lutte contre les ravageurs ou les maladies des cultures ou pour la fertilisation des sols, caractérisé en ce qu'il comprend la culture directe par fermentation à l'état solide dudit microorganisme sur au moins un support solide en particules, insoluble dans le milieu de fermentation et la récupération desdites particules qui sont colonisées par le microorganisme, la maturité de la culture étant contrôlée Pour permettre au microorganisme de sporuler dans les conditions d'épandage.

19. Procédé selon la revendication 18, caractérisé en ce que le microorganisme est un champignon.

20. Procédé selon la revendication 19 caractérisé en ce que l'on arrête la fermentation sensiblement au stade de l'obtention du mycélium, ce qui conduit à un produit dit immature.

21. Procédé selon la revendication 19, caractérisé en ce que l'on poursuit la fermentation jusqu'au stade de l'obtention de mycélium et de spores, ce qui conduit à un produit dit mature.

22. Procédé selon l'une quelconque des revendications 19 à 21, caractérisé en ce que le champignon utilisé est un champignon entomopathogène, efficace contre un ou des insectes ravageurs des cultures.

23. Procédé selon l'une quelconque des revendications 19, 20 et 22, caractérisé en ce que le champignon est Beauveria bassiana et la fermentation a lieu durant 20 à 60 heures, et de préférence 48 heures auquel cas on obtient un produit immature.

24. Procédé selon l'une quelconque des revendications 19, 21 et 22, caractérisé en ce que le champignon est Beauveria bassiana et la fermentation a lieu durant au minimum 90 heures, auquel cas on obtient un produit mature.

25. Procédé selon l'une quelconque des revendications 18 à 24, caractérisé en ce que lesdites particules ont été ou non préalablement stérilisées.

26. Procédé de traitement des végétaux contre des maladies ou les ravageurs, caractérisé en ce qu'on épand une quantité efficace du produit selon l'une quelconque des revendications 1 à 13, 16 et 17 sur les végétaux à traiter.

27. Procédé selon la revendication 26 caractérisé en ce qu'on traite les végétaux contre Ostriania nubilalis qui est la pyrale du maïs.

28. Procédé de fertilisation des sols, caractérisé en ce qu'on épand une quantité efficace du produit selon l'une quelconque des revendications 1 à 11 et 17, sur les sols à traiter.

## Claims

1. Agricultural treatment product based on at least one microorganism useful for controlling crop pests or diseases or for soil fertilization, characterized in that it comprises particles formed from at least one solid support carrying the microorganism, compatible with a mechanized application and in that it can be spread in the field directly after it is obtained by direct culture and solid state fermentation on said particles, the particles being precolonized by said microorganisms during the fermentation, while the maturity of the culture of microorganism is controlled.

2. Product according to claim 1, characterized in that the microorganism is a fungus.

3. Product according to claim 2, characterized in that the fungus is entirely in mycelium form at the time of spreading, in which case said product is considered immature.

4. Product accordingt to claim 2, characterized in that the fungus is in the form of mycelium and spores, in which case said product is considered mature.

5. Product according to claim 2, characterized in that it is composed of a mixture of immature and mature forms.

6. Product according to any of claims 1 to 5, characterized in that the said support has a density comprised preferentially between 0.6 and 1.

7. Product according to any of claims 1 to 6, characterized in that said support is of inorganic or organic origin.

8. Product according to claim 7, characterized in that said support is montmorillonite.

9. Product according to claim 7, characterized in that said support is attapulgite.

10. Product according to any of claims 1 to 9, characterized in that said support has a minimum water retention capacity of 40%.

11. Product according to any of claims 1 to 10, characterized in that said particles are of a size comprised between 100 and 1000 µm, preferentially around 500 µm.

12. Product according to any of claims 2 to 11, characterized in that the fungus used is an entomopathogenic fungus, effective against one or more crop insect pests.

13. Product according to ally of claims 2 to 12, characterized in that the fungus is Beauveria bassiana.

14. Strain of Beauveria bassiana deposited in the National Collection of Microorganism Cultures under N° I-867 and used in the product according to any of claims 1 to 13.

15. Strain of Beauveria bassiana ,characterized in that it results from one or more mutations of the strain according to claim 14.

16. Product according to claim 13, characterized in that the Beauveria bassiana strain is a strain according to any of claims 14 and 15.

17. Product according to any of claims 1 to 13, characterized in that it is in dry form for storing and spreading.

18. Process for preparing a product for agricultural treatment based on at least one microorganism for controlling crop pests or diseases or for soil fertilization, characterized in that it comprises direct growth by solid state fermentation of said microorganism on at least one solid support in particle form, insoluble in the fermentation medium, and the recovery of said particles which are colonized by the microorganism, maturing of the culture being controlled to allow the microorganism to sporulate under spreading conditions.

19. Process according to claim 18, characterized in that the microorganism is a fungus.

20. Process according to claim 19, characterized in that the fermentation is continued until mycelium is obtained, resulting in a so-called immature product.

21. Process according to claim 19, characterized in that the fermentation is continued until mycelium and spores are obtained resulting in a so-called mature product.

22. Process according to any of claims 19 to 21, characterized in that the fungus used is an entomopathogenic fungus, effective against one or several crop insect pests.

23. Process according to any of claims 19, 20 and 22, characterized in that the fungus is Beauveria bassiana and fermentation takes place over 20 to 60 hours, and preferably 48 hours, in which case an immature product is obtained.

24. Process according to any of claims 19, 21 and 22, characterized in that the fungus is Beauveria bassiana and fermentation takes place over a minimum of 90 hours, in which case a mature product is obtained.

25. Process according to any of claims 18 to 24, characterized in that said particles have or have not been previously sterilized.

26. Process of treating plants against diseases or pests, characterized in that an effective quantity of the product according to any of claims 1 to 13, 16 and 17 is spread on plants to be treated.

27. Process according to claim 26, characterized in that plants are treated against Ostriania nubilalis, known as corn pyrale.

28. Soil fertilization process, characterized in that an effective quantity of the product according to any of claims 1 to 11 and 17 is spread on the soils to be treated.

## Patentansprüche

1. Produkt zur agronomischen Behandlung auf der Basis von wenigstens einem Mikroorganismus, das sich zur Bekämpfung von Schädlingen oder von Krankheiten der Kulturen oder zur Bodendüngung eignet, dadurch gekennzeichnet, daß es Teilchen umfaßt, die aus einem festen Träger gebildet sind, der den Mikroorganismus trägt und mit einer mechanisierten Auftragung verträglich ist, und daß es direkt nach seiner Gewinnung durch direkte Kultur und Fermentation in festem Zustand auf den Teilchen auf dem Feld verteilt werden kann, wobei die Mikroorganismen während der Fermentation die Teilchen im voraus besiedeln und die Reife der Kultur des Mikroorganismus kontrolliert wird.

2. Produkt gemäß Anspruch 1, dadurch gekennzeichnet, daß der Mikroorganismus ein Pilz ist.

3. Produkt gemäß Anspruch 2, dadurch gekennzeichnet, daß der Pilz zum Zeitpunkt des Verteilens zur Gänze in Form des Mycels vorliegt, in welchem Falle das Produkt als unreif bezeichnet wird.

4. Produkt gemäß Anspruch 2, dadurch gekennzeichnet, daß der Pilz in Form des Mycels und von Sporen vorliegt, in welchem Falle das Produkt als reif bezeichnet wird.

5. Produkt gemäß Anspruch 2, dadurch gekennzeichnet, daß es aus einem Gemisch der reifen und der unreifen Form besteht.

6. Produkt gemäß einem der Ansprüche 1 bis 5, dadurch gekennzeichnet, daß der Träger eine Dichte hat, die vorzugsweise zwischen 0,6 und 1 liegt.

7. Produkt gemäß einem der Ansprüche 1 bis 6, dadurch gekennzeichnet, daß der Träger mineralischen oder organischen Ursprungs ist.

8. Produkt gemäß Anspruch 7, dadurch gekennzeichnet, daß es sich bei dem Träger um Montmorillonit handelt.

9. Produkt gemäß Anspruch 7, dadurch gekennzeichnet, daß es sich bei dem Träger um Attapulgit handelt.

10. Produkt gemäß einem der Ansprüche 1 bis 9, dadurch gekennzeichnet, daß der Träger ein Wasserrückhaltevermögen von wenigstens 40% besitzt.

11. Produkt gemäß einem der Ansprüche 1 bis 10, dadurch gekennzeichnet, daß die Teilchen eine Größe zwischen 100 und 1000 µm und vorzugsweise eine Größe von ungefähr 500 µm haben.

12. Produkt gemäß einem der Ansprüche 2 bis 11, dadurch gekennzeichnet, daß der verwendete Pilz ein entomopathogener Pilz ist, der gegen ein oder mehrere Insekten, die die Kulturen schädigen, wirksam ist.

13. Produkt gemäß einem der Ansprüche 2 bis 12, dadurch gekennzeichnet, daß es sich bei dem Pilz um *Beauveria bassiana* handelt.

14. Stamm von *Beauveria bassiana*, der bei der Collection Nationale de Cultures de Microorganismes unter der Nr. I 867 hinterlegt wurde und im Produkt gemäß einem der Ansprüche 1 bis 13 verwendet wird.

15. Stamm von *Beauveria bassiana*, dadurch gekennzeichnet, daß er sich durch eine oder mehrere Mutationen aus dem Stamm gemäß Anspruch 14 ergibt.

16. Produkt gemäß Anspruch 13, dadurch gekennzeichnet, daß es sich bei dem *Beauveria-bassiana-*Stamm um einen Stamm gemäß einem der Ansprüche 14 und 15 handelt.

17. Produkt gemäß einem der Ansprüche 1 bis 13 und 16, dadurch gekennzeichnet, daß es zur Lagerung und Verteilung in trockener Form vorliegt.

18. Verfahren zur Gewinnung eines Produkts zur agronomischen Behandlung auf der Basis von wenigstens einem Mikroorganismus zur Bekämpfung von Schädlingen oder von Krankheiten der Kulturen oder zur Bodendüngung, dadurch gekennzeichnet, daß es die direkte Kultur des Mikroorganismus durch Fermentation in festem Zustand auf wenigstens einem aus Teilchen bestehenden festen Träger, der im Fermentationsmedium unlöslich ist, und die Gewinnung der von dem Mikroorganismus besiedelten Teilchen umfaßt, wobei die Reife der Kultur kontrolliert wird, so daß der Mikroorganismus unter den Bedingungen der Verteilung Sporen bilden kann.

19. Verfahren gemäß Anspruch 18, dadurch gekennzeichnet, daß der Mikroorganismus ein Pilz ist.

20. Verfahren gemäß Anspruch 19, dadurch gekennzeichnet, daß die Fermentation ungefähr im Stadium des Erhaltens des Mycels unterbrochen wird, was zu einem als unreif bezeichneten Produkt führt.

21. Verfahren gemäß Anspruch 19, dadurch gekennzeichnet, daß die Fermentation bis zum Stadium des Erhaltens des Mycels und von Sporen fortgeführt wird, was zu einem als reif bezeichneten Produkt führt.

22. Verfahren gemäß einem der Ansprüche 19 bis 21, dadurch gekennzeichnet, daß der verwendete Pilz ein entomopathogener Pilz ist, der gegen ein oder mehrere Insekten, die die Kulturen schädigen, wirksam ist.

23. Verfahren gemäß einem der Ansprüche 19, 20 und 22, dadurch gekennzeichnet, daß es sich bei dem Pilz um *Beauveria bassiana* handelt und die Fermentation 20 bis 60 Stunden lang und vorzugsweise 48 Stunden lang erfolgt, in welchem Fall man ein unreifes Produkt erhält.

24. Verfahren gemäß einem der Ansprüche 19, 21 und 22, dadurch gekennzeichnet, daß es sich bei dem Pilz um *Beauveria bassiana* handelt und die Fermentation wenigstens 90 Stunden lang erfolgt, in welchem Fall man ein reifes Produkt erhält.

25. Verfahren gemäß einem der Ansprüche 18 bis 24, dadurch gekennzeichnet, daß die Teilchen vorher sterilisiert worden sind oder nicht sterilisiert worden sind.

26. Verfahren zur Behandlung von Pflanzen gegen Krankheiten oder Schädlinge, dadurch gekennzeichnet, daß man eine wirksame Menge des Produkts gemäß einem der Ansprüche 1 bis 13, 16 und 17 auf die zu behandelnden Pflanzen verteilt.

27. Verfahren gemäß Anspruch 26, dadurch gekennzeichnet, daß man die Pflanzen gegen *Ostriania nubilalis*, den Maiszünsler, behandelt.

28. Verfahren zur Bodendüngung, dadurch gekennzeichnet, daß man eine wirksame Menge des Produkts gemäß einem der Ansprüche 1 bis 11 und 17 auf die zu behandelnden Böden verteilt.
